(19) Europäisches Patentamt / European Patent Office / Office européen des brevets

(11) **EP 3 027 581 B1**

(12) **EUROPEAN PATENT SPECIFICATION**

(45) Date of publication and mention
of the grant of the patent:
**16.05.2018 Bulletin 2018/20**

(21) Application number: **14744335.2**

(22) Date of filing: **28.07.2014**

(51) Int Cl.:
*C07B 35/06* (2006.01)          *C07C 17/25* (2006.01)
*C07C 21/06* (2006.01)

(86) International application number:
**PCT/EP2014/066156**

(87) International publication number:
**WO 2015/014783 (05.02.2015 Gazette 2015/05)**

(54) **PROCESS FOR THE DEHYDROHALOGENATION OF A HALOGENATED ORGANIC NON POLYMERIC COMPOUND**

VERFAHREN ZUR DEHYDROHALOGENIERUNG EINER HALOGENIERTEN, ORGANISCHEN, NICHTPOLYMEREN VERBINDUNG

PROCÉDÉ POUR LA DÉSHYDROHALOGÉNATION D'UN COMPOSÉ ORGANIQUE HALOGÉNÉ NON POLYMÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2013 EP 13178903**

(43) Date of publication of application:
**08.06.2016 Bulletin 2016/23**

(73) Proprietor: **Solvay SA**
**1120 Bruxelles (BE)**

(72) Inventors:
• **DE VOSS, Dirk**
**3220 Holsbeek (BE)**
• **BOUDEWIJNS, Tom**
**3001 Heverlee (BE)**
• **PICCININI, Marco**
**1120, Neder-over-Heembeek**
**Brussels (BE)**
• **LIEBENS, Armin T.**
**1200 Brussels (BE)**

(56) References cited:
**US-A- 2 322 258     US-A- 2 877 277**

• **S.S. SHAVANOV, ET AL.: "Synthesis and properties of triethylbenzylammonium alkoxides, reactivity in elimination and nucleophilic substitution reactions", JOURNAL OF ORGANIC CHEMISTRY OF THE USSR, vol. 26, no. 5, 1990, pages 643-647, XP008166896, Consultants Bureau, New York, NY, US ISSN: 0022-3271 cited in the application**
• **S. GRINBERG, ET AL.: "Catalytic activity of PEG-quat phase-transfer catalysts in dehydrohalogenation reactions", TETRAHEDRON, vol. 47, no. 16-17, April 1991 (1991-04), pages 2895-2902, XP055098311, Elsevier Science Publishers, Amsterdam, NL ISSN: 0040-4020, DOI: 10.1016/S0040-4020(01)87094-0**
• **TAO ZHAO, ET AL.: "A highly efficient approach for dehydrochlorinating polyvinyl chloride: catalysis by 1-butyl-3-methylimidazolium chloride", GREEN CHEMISTRY, vol. 12, no. 6, 12 May 2010 (2010-05-12), pages 1062-1065, XP055098300, ISSN: 1463-9262, DOI: 10.1039/b927106f cited in the application**

EP 3 027 581 B1

## Description

The present invention relates to a process for the dehydrohalogenation of a halogenated organic non polymeric compound, preferably a chlorinated organic non polymeric compound like DCE (1,2-dichloroethane).

Current industrial processes utilize high temperature pyrolysis for cracking DCE into vinyl chloride non polymeric compound (VCM) and HCl. However, said process is characterized by some important drawbacks : on the one hand, DCE conversion is limited at low temperature but on the other hand, increasing temperature leads to by-products and coke formation so that in practice, an optimal temperature of about ~500°C is used industrially, which is energy consuming and still leads to limited conversion (of about 50-60 %) and selectivity around 98 %.

Similar problems are generally encountered with other halogenated organic non polymeric compounds, particularly chlorinated ones so that several attempts have been made in the past to replace pyrolysis by a catalyzed chemical reaction. So have been disclosed in literature, dehydrochlorination reactions using alkali metal chlorides, molten salts, $Mg(OH)_2$, the acid sites of oxide catalysts and also liquids like triethylbenzylammonium alkoxides. However, none of these provides all together high conversion and selectivity at low temperature and an environmentally friendly character. By "low temperature" is generally meant a temperature below 300°C.

On the other hand, recently, ionic liquids (ILs), i.e., organic salts in "melted" (or liquid) state have been used to dehydrochlorinate polyvinylchloride polymers (PVC). These ILs are environmentally friendly due to their chemical and thermal stability, low vapor pressure and ease of recycling, and their ability to effectively dehydrochlorinate PVC has been disclosed for instance by Zhao & al, "Dehydrochlorination of polyvinyl chloride in basic ionic liquids", the 5th ISFR October 11-14, 2009, Chengdu, China, 260-262; "A highly efficient approach for dehydrochlorinating polyvinyl chloride catalysis by 1-butyl-3-methylimidazolium chloride", Green Chem., 2010, 12, 1062-1065 and by Glas & al. "End-of-life treatment of PVC and chlorinated polyethylene by dehydrochlorination in Ionic Liquids", Chem Sus Chem 2014, 7 (2) 610-7.

In fact, dehydrochlorination (and more generally: dehydrohalogenation) of a polymer like PVC is rather easy because during this process, double conjugated double bonds are formed which are thermodynamically favored.

The Applicant has now found that surprisingly, some ILs can be used to dehydrochlorinate DCE as a replacement of its pyrolysis or caustic treatment, and more generally to dehydrohalogenate halogenated non polymeric compounds instead of pyrolysing them. Dehydrohalogenation of non polymeric compounds is in fact less favored thermodynamically since no conjugated double bonds are formed during the process.

Therefore, the present invention concerns a process for the dehydrohalogenation of a halogenated organic non polymeric compound using a catalyst comprising an ionic liquid having the formula :

$$[Cat^+][X^-]$$

Wherein : $[Cat^+]$ represents one or more organic cationic species, and
$[X^-]$ represents one or more anionic species.

By "catalyst" is meant a compound which is participating in the reaction, but not stoichiometrically changed in it i.e. which is not consumed by the reaction. In that regard, the alkoxide compound used in the dehydrohalogenation reaction described in S.S. Shavanov, et al., Journal of Organic Chemistry of the USSR, 1990, 26(5), 643-647 (XP008166896), namely triethylbenzylammonium ethoxide, cannot be seen as a catalyst because it is not a compound that is not consumed by the reaction but instead, it participates to the stoichiometry of the reaction, which is basically an acid-base reaction. This can be seen from the 4th paragraph of page 644 where it is specified that a stoichiometric amount of alkoxide is used.

By "ionic liquid" is meant an organic salt which is liquid at the temperature used for the desired reaction and preferably, has a melting temperature of at least 10°C below the temperature used for the desired reaction.

The terms "a catalyst comprising an ionic liquid" do not only imply that the catalyst must not be the pure IL but also, that the IL may be incorporated in the catalyst, which may hence be solid. For example, the IL may be impregnated in/on a carrier as will be explained more in detail below. Also, the IL may be a monomer which is polymerized. An example thereof is poly-diallyl-dimethylammonium chloride (shortened polyDADMAC or polyDDA) which is a homopolymer of diallyldimethylammonium chloride (DADMAC).

Since many ILs are corrosive in the reaction conditions of the invention, when they are used on a support, it might be advantageous to choose a solid carrier like silica, alumina, titania, zirconia or another stable oxide.

The IL can also be used as a mixture with an inert compound (which does not react in the reaction conditions of the invention) or with the reagent i.e. with the dehydrohalogenated compound. In that case, the IL concentration in the mixture is preferably at least 5 %, preferably at least 10 % molar.

According to the invention, the cation $[Cat^+]$ is an organic cation which may for instance be of the phosphonium or ammonium type, or also of heteroaromatic nature.

The cation [Cat$^+$] can be a phosphonium ion with the following general formula : [PRaRbRcRd]$^+$, wherein Ra, Rb, Rc, Rd can be independently chosen to be alkyl (with C1 till C30), aryl (C6 to C10), arylalkyl, heteroaryl, heteroarylalkyl or hydrogen; whereby the alkyl, aryl, arylalkyl, heteroaryl, heteroarylalkyl groups can be substituted with (cyclo)alkyl, (cyclo)alkoxy, aryl, arylalkyl, amide, ammonium or phosphonium groups.

The cation [Cat$^+$] can also be an ammonium ion of the type [NRaRbRcRd]$^+$ wherein Ra, Rb, Rc, Rd can be independently chosen to be alkyl (with C1 till C30), aryl (C6 to C10), arylalkyl, heteroaryl, heteroarylalkyl or hydrogen; whereby the alkyl, aryl, arylalkyl, heteroaryl, heteroarylalkyl groups can be substituted with (cyclo)alkyl, (cyclo)alkoxy, aryl, arylalkyl, amide, ammonium or phosphonium groups,

The cation [Cat$^+$] can also be of heteroaromatic nature, with N or P atoms in the aromatic ring, whereby the ring can be of the imidazolium, pyridinium, pyrrolidinium, benzimidazolium, phthalazinium, piperidinium, pyrrolium, quinazolinium, quinolinium, iso-quinolinium, indolinium or indolium type.

Preferably, the cation is of the phosphonium type [PRaRbRcRd]$^+$. It has been found that ILs with such a cation show superior thermal stability under the dehydrohalogenation conditions. Preferably, the substituents Ra, Rb, Rc, Rd of this phosphonium cation are independently selected from linear or branched alkyl, aryl, arylalkyl groups consisting exclusively of C and H atoms, and hydrogen.

Preferred phosphonium type cations include but are not limited to tetrabutylphosphonium or Bu$_4$P$^+$ or [P4,4,4,4] or C4444P+Cl-; tributylphosphonium or HBu$_3$P$^+$ or [PH4,4,4] or HC444PCl-; tributyl(tetradecyl)phosphonium or (C$_{14}$H$_{29}$)Bu$_3$P$^+$ or [P14,4,4,4] or C44414P+Cl- and trihexyl(tetradecyl)phosphonium or (C$_{14}$H$_{29}$)Hex$_3$P+ or [P14,6,6,6] or C66614P+Cl-.

Bu$_4$P$^+$ or [P4,4,4,4] is preferred because it is efficient and readily commercially available, namely under the brand name Cyphos® 443P from the company Cytec. It has been found that short chain substituents improve the solvent performances of the ILs so that shorter substituents of P+ like methyl, ethyl or propyl might lead to better results. Unfortunately, such products are not readily commercially available today.

The anion [X$^-$] is preferably a halide and even more preferably the same halide as the one to be eliminated by dehydrohalogenation from the organic compound in order to avoid any anion exchange that will always occur due to thermodynamic equilibrium. Hence, in the case of dehydrochlorination, the cation is preferably a chloride.

In the present invention, the catalyst may comprise mixtures of ionic liquids as described in the above. In order to increase the charge density around the phosphonium ion, salts may be added to the ionic liquid and preferably salts having again the same anion as the one to be eliminated by dehydrohalogenation from the organic compound. In the case of dehydrochlorination reactions, it has been found that the addition of CsCl to the IL improved its thermal stability. A similar effect can be obtained with other cesium or potassium salts such as KCl, Cs2CO3, K2CO3, CsOH or KOH.

In the frame of the present invention, by "non polymeric compound" is meant a molecule of low molecular weight having only limited repetitions (typically maximum 3) of the same chemical unit, by opposition to a polymer. Hence, it is generally a liquid or a gas. It is preferably a molecule having a chemical structure such that by dehydrohalogenating it, non-conjugated double bonds are formed. Preferably, said non polymeric compound comprises a single chemical unit without any repetition.

The non polymeric compounds that can be dehydrohalogenated by the process of the invention comprise, but are not limited to, chlorinated, fluorinated and brominated non polymeric compounds, preferably chlorinated ones, more preferably TCE (trichloroethane) or DCE. Especially DCE can easily be dehydrochlorinated to VCM using the process of the invention.

Parameters that should be optimized in the reaction of the invention and which may also depend on the nature of the non polymeric compound comprise, but are not limited to : nature and amount of the ionic liquid; the fact that it is supported or not; temperature; pressure; reactor geometry/type; contact time and exchange interface between the non polymeric compound and IL; agitation of the reaction medium.

The temperature of reaction is generally as high as possible considering the thermal stability of the IL used. In the case of phosphonium based ILs, the reaction temperature is advantageously higher than or equal to 180°C, preferably to 200 and even more preferably to 240°C. Preferably, the temperature is lower than or equal to 280°C, more preferably to 260°C.

The volumetric concentration of the halogenated organic compound in the feed (incoming stream) is preferably equal to or larger than 2 vol %. More advantageously, the volumetric concentration is equal to or larger than 5 vol % in the incoming stream.

Suitable industrial reactors for carrying out this reaction comprise but are not limited to CSTR type of reactor, PFR type of reactor, falling film reactors...

Since ILs are corrosive, the reactors listed above should be designed to allow getting very low corrosion during a normal industrial life time (several years, typically at least 10 years). They are preferably based on a corrosion resistant material either in massive form or as a protective layer allowing the apparatus to resist to the corrosivity of the reaction medium.

Examples of materials which are resistant to halogenated acids, preferably to HCl, in dissociated form, that can be used either in massive form or as protective layer are metals, fluorinated polymers, ceramics, (impregnated) graphite, enamel and silicon carbide.

Examples of materials that may be used as protective layer (made of a different material than the one providing the mechanical resistance) are metals such as Nb, Ta, metal alloys such as Hastelloy® C276, Hastelloy® HB2, Monel®, Inconnel®, Incoloy®, enamels such as Pfaudler®email WWG, Pfaudler®email 4300, Pfaudler®email ASG, fluorinated polymers like PTFE, PFA, MFA, PVDF...

For metals, the corrosion allowance (or amount of corrosion allowed before having to replace the concerned piece) is usually less than 5 mm, a corrosion allowance of less than 3 mm being preferable, a corrosion allowance of less than 2 mm being more preferable and a corrosion allowance of less than 1.8 mm being particularly preferred. Corrosion allowance for metals is usually higher than 0.01 mm, preferably higher than 0.03 mm and more preferably higher than 0.05 mm.

For non-metallic protective layers, the thickness of the layer is usually greater than 0.1 mm, preferably greater than 0.3 mm, more preferably greater than 0.5 mm. Usually the thickness of the protective layer is lower than 20 mm, preferably lower than 15 mm and more preferably lower than 10 mm.

The apparatus could be made in a single material or be bimaterial. When the apparatus is bimaterial, generally the support material is not in contact with the reaction medium and provides the mechanical resistance. The support material could be metallic or plastic. Example of support materials are carbon steel, stainless steel, fiber glass reinforced polyester, polyethylene, polypropylene, PVC... Carbon steel is preferred. The support material providing the mechanical resistance is usually subjected to the corrosivity of the external atmosphere. Preferably the support material also has a protective layer versus the external corrosion. External protective layers could be an extra thickness of the same material, or a paint layer. When the protective layer is an extra thickness it is usually higher than 0.1 mm, preferably higher than 0.3, and more preferably higher than 0.5 mm, while it usually is lower than 5 mm, preferably lower than 3 mm, more preferably lower than 2.5 mm.The present invention will now be illustrated by means of the Examples described below and of which the results are attached to the present specification.

**Examples**

Experiments were carried out using two different lab bench reactors.

Reactor (1): A given amount of the chosen ionic liquid was weighed and placed in a glass reactor vial (if needed, the ionic liquid may have been previously dried using a Schlenk-line for 3 hours). In some experiments, other additives were also added to the ionic liquid (e.g. $ZnCl_2$, $HC444P^+Cl^-$...).

The reactor was placed in an aluminum heating block and heated up to the desired reaction temperature (180, 200, 220 or 240°C) and flushed with dry $N_2$ as additional drying step. Such flow of $N_2$ was always kept constant throughout the experiment for dilution and safety purposes. The reaction mixture was agitated by means of a magnetic stirring bar placed at the bottom of the reactor with a rotating speed of at least 200 rpm.

DCEa was fed to the reactor using two different methods:

(1a) The reaction was then started by flushing additional 5 mL/min of dried N2 into a DCEa saturator prior entering the reactor. This accounted for a DCEa inlet flow of about 22 $\mu$mol/min (calculated for T = 25°C, p = 1 atm and assuming complete liquid-gas equilibrium, noting that flow slightly varies due to daily pressure and temperature changes of the laboratory room).

(1b) The reaction was started by addition of DCEa into the reactor using a perfusion pump that allow a DCEa flow rate range of 1 up to 45 mmol/h. Although method (1b) represents a closer set-up to industrial application, due to specific laboratory reactor design, a higher fluctuation in experimental results was observed.

Reactor (2): a horizontal coiled glass tube reactor was used (I.D. 4 - 5 mm and total length of 900 -110 mm). A given amount of the chosen ionic liquid was weighed and placed in the reactor via an 'evaporation step' (if needed, the ionic liquid may have been previously dried using a Schlenk-line for 3 hours). The desired amount of IL was dissolved in about 10 mL of DMSO. Then, the reactor was completely filled with the DMSO-IL solution. DMSO was then evaporated away by gently heating the reactor at a temperature of about 50°C. After this procedure and depending on the amount of IL used, about 1 up to 15 vol% of the reactor volume was filled with IL. The IL was evenly and homogeneously distributed throughout the reactor with only minor flaws. The reactor was then buried into a sand bath. The reactor was then flushed with N2 to ensure removal of the DMSO. The reactor temperature was then brought to the desired one by using a commercial heating plate. This type of reactor is of particular importance as it was designed to reproduce in lab scale those industrial reactors that feature high gas-liquid surface exchange such as falling film reactor. DCEa was fed to the reactor using two different methods:

(2a) The reaction was then started by flushing additional 5 mL/min of dried N2 into a DCEa saturator prior entering the reactor. This accounted for a DCEa inlet flow of about 24 $\mu$mol/min (flow slightly varied due to daily pressure and temperature changes of the laboratory room).

(2b) The reaction was started by addition of DCEa into the reactor using a perfusion pump that allow a DCEa flow rate range of 1 up to 45 mmol/h.

The reaction was carried out for the required time and out-let gases were analyzed with the help of an on-line GC.

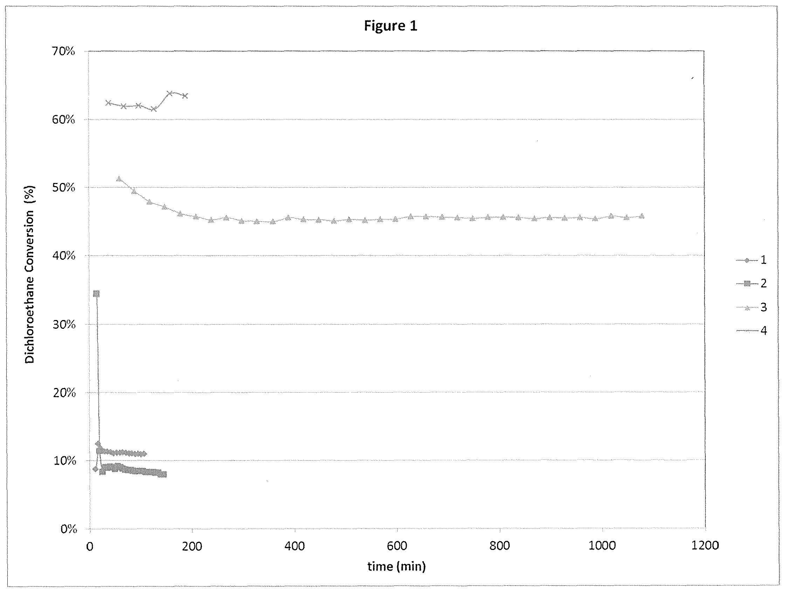

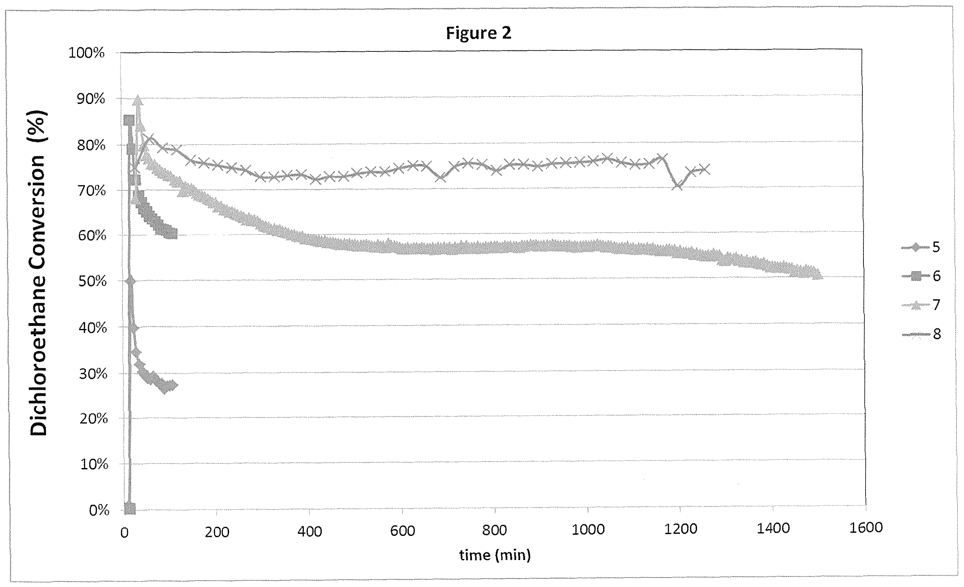

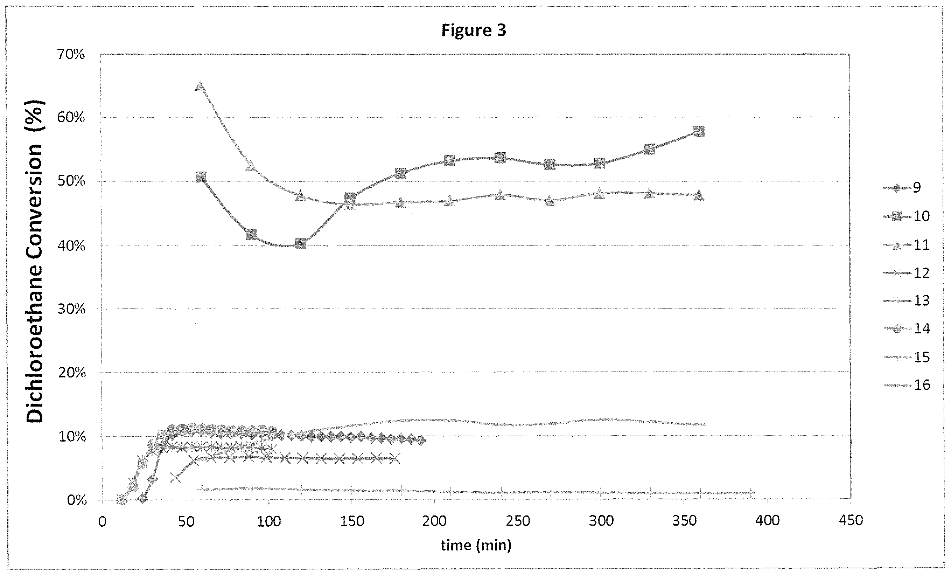

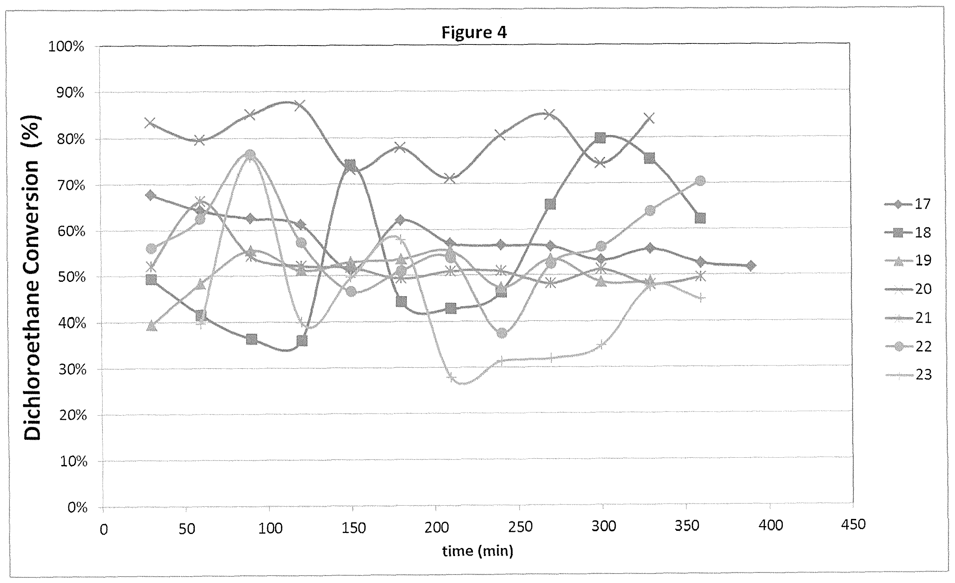

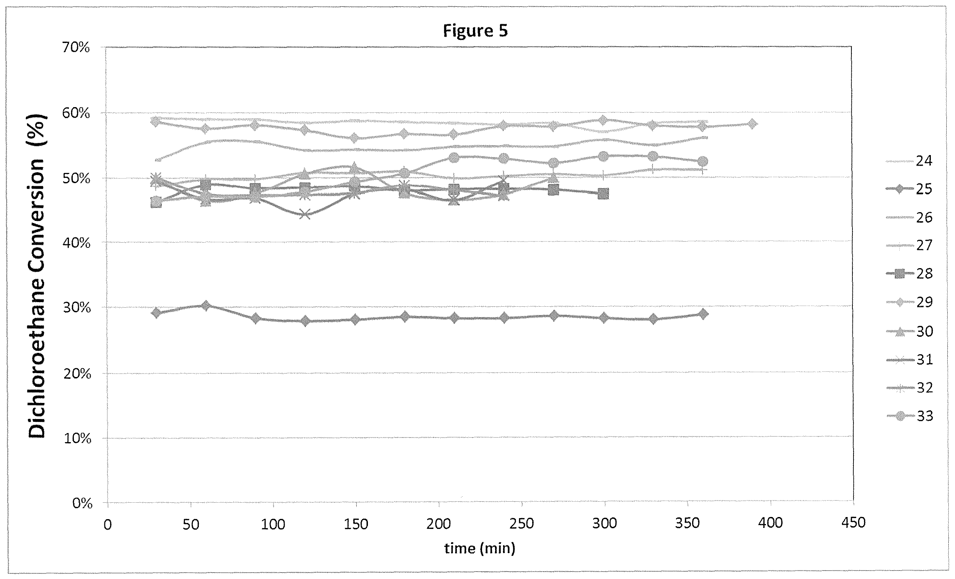

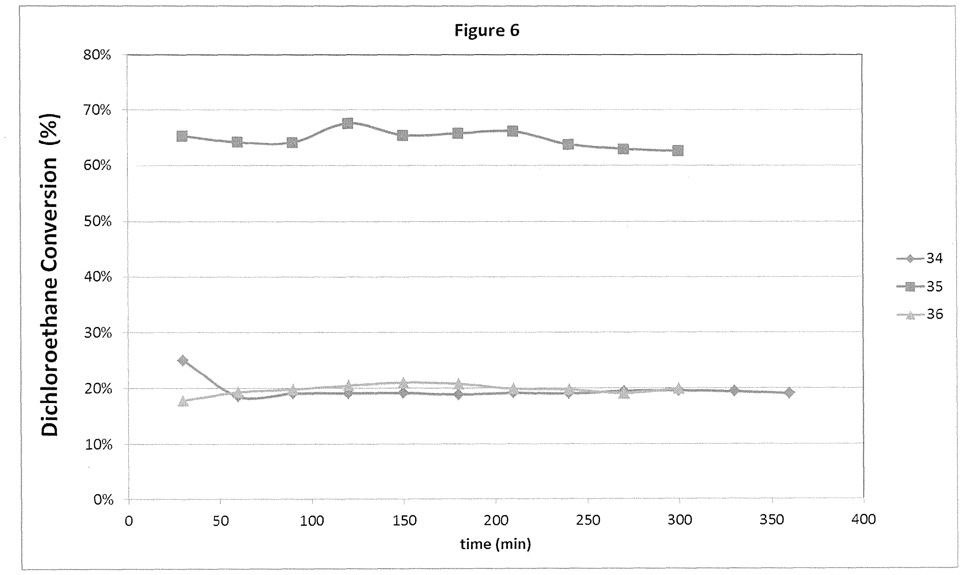

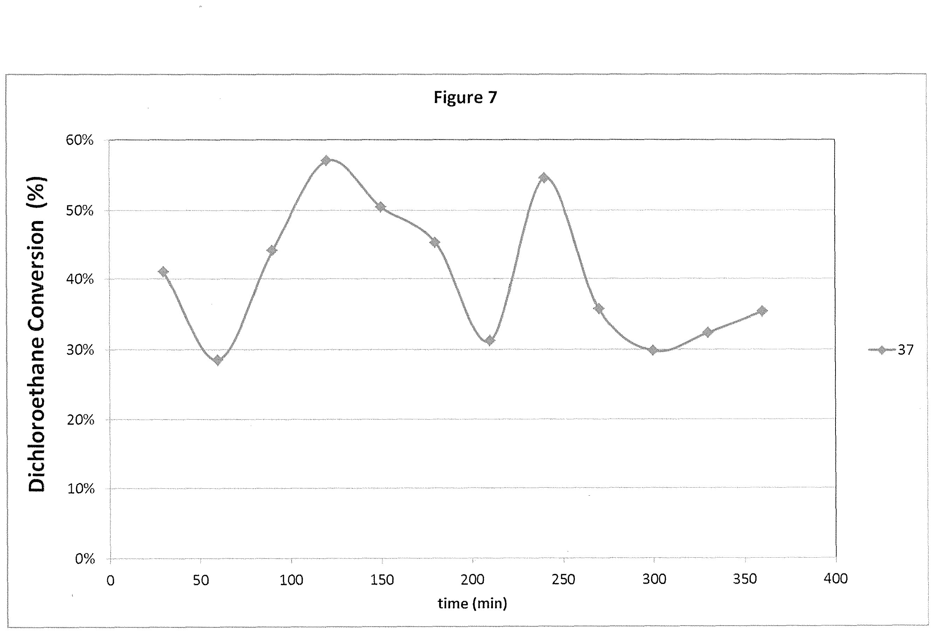

The detailed experimental conditions are shown in Table 1 below and the results of the trials are shown in Figures 1 to 7 attached. There, conversion is plotted as function of time. Conversion was calculated as follows:

$$\chi_{DCE} = \frac{DCEa_{inlet} - DCEa_{outlet}}{DCEa_{inlet}} * 100$$

where $DCEa_{inlet}$ and $DCEa_{outlet}$ are the flow rates of the DCEa before and after the reactor respectively expressed in mol/h. While $DCEa_{outlet}$ was measured with the help of the online GC, $DCEa_{inlet}$ was only measured before and after the reactor by using a by-pass valve.

In all the experiments an initial decrease in the apparent conversion may be observed due to the initial dissolution of DCE in the IL. Such dissolution period lasts in average about 60 min after which, real catalytic conversion is measured. In all the experiments, selectivity was evaluated on the basis of the amount of by-products formed and analyzed at the GC. It was estimated that in all the cases, selectivity was > 95 %.

Surprisingly, all the systems tested displayed a stable conversion at high selectivity.

The series comprising experiments 2, 3, 4 highlight the effect of temperature on DCEa conversion. Results show that increasing the temperature leads to an increase in conversion.

The series comprising experiments:

| | |
|---|---|
| 2, 1, 5 | (Tetrabutyl phosphonium chloride, 180°C) |
| 6, 7 | (Tetrabutyl phosphonium chloride, 200°C) |
| 4, 8 | (Tetrabutyl phosphonium chloride, 240°C) |
| 12, 13, 14 | (Trihexyl (tetradecyl) phosphonium chloride, 180°C) |

highlight the effect of the amount of IL used on conversion. It is noteworthy that the amount of IL utilized is correlated to reaction residence time. Results show that increasing the amount of IL is beneficial for improving DCEa conversion.

Experiments 9, 22 and 23 show the influence of the addition of inorganic salts to the reaction mixture (Experiment 9 should be compared to experiment 1 whilst experiments 22 and 23 should be compared to experiment 18).

Experiments 10 and 11 were carried out using a mixture of two different ionic liquids.

Different ILs have been tested and results are shown by experiments 15, 16, 24, 25, 26, 27, 28, 29, 30, 31, 32, 33.

The results acquired with the reactor set-up previously defined as (1b) are shown in experiments 17, 18, 19, 20, 21, 22, 23. In particular, experiments 17, 18, 19, 21 show the effect of DCEa inlet flow rate on DCEa conversion.

Experiments 34, 35, 36 were acquired using the reactor set-up previously defined as (2a).

Experiment 37 was acquired using the reactor set-up previously defined as (2b). In this case, a higher fluctuation in experimental results was observed.

Table 1

| Figure | Experiment (n°) | Ionic Liquid used | | Amount (g) | Additive | | Amount (g) | Reactor type | Temperature (°C) | N2 flow (mL/min) | N2 flow (diluent) (mL/min) | DCE flow (mmol/h) |
|---|---|---|---|---|---|---|---|---|---|---|---|---|
| | | | Name | | | Name | | | | | | |
| Figure 1 | 1 | C4444P+Cl- | Tetrabutyl phosphonium chloride | 3,3 | | | | 1a | 180 | 5 | 5 | 1,4 |
| | 2 | C4444P+Cl- | Tetrabutyl phosphonium chloride | 1,8 | | | | 1a | 180 | 5 | 5 | 1,4 |
| | 3 | C4444P+Cl- | Tetrabutyl phosphonium chloride | 1,8 | | | | 1a | 220 | 5 | 5 | 1,4 |
| | 4 | C4444P+Cl- | Tetrabutyl phosphonium chloride | 1,8 | | | | 1a | 240 | 5 | 5 | 1,4 |
| Figure 2 | 5 | C4444P+Cl- | Tetrabutyl phosphonium chloride | 5,6 | | | | 1a | 180 | 5 | 5 | 1,4 |
| | 6 | C4444P+Cl- | Tetrabutyl phosphonium chloride | 5,6 | | | | 1a | 200 | 5 | 5 | 1,4 |
| | 7 | C4444P+Cl- | Tetrabutyl phosphonium chloride | 8,6 | | | | 1a | 200 | 5 | 5 | 1,4 |
| | 8 | C4444P+Cl- | Tetrabutyl phosphonium chloride | 2,1 | | | | 1a | 240 | 5 | 5 | 1,4 |
| Figure 3 | 9 | C4444P+Cl- | Tetrabutyl phosphonium chloride | 3,3 | ZnCl2 | Zinc Chloride | 0,15 | 1a | 180 | 5 | 5 | 1,4 |
| | 10 | C4444P+Cl- | Tetrabutyl phosphonium chloride | 0,9 | HC444P+Cl- | Tributyl phosphonium chloride | 0,69 | 1a | 240 | 5 | 5 | 1,4 |
| | 11 | C4444P+Cl- | Tetrabutyl phosphonium chloride | 2,0 | HC444P+Cl- | Tributyl phosphonium chloride | 0,74 | 1a | 240 | 5 | 5 | 1,4 |
| | 12 | C666C14P+Cl- | Trihexyl (tetradecyl) phosphonium chloride | 3,1 | | | | 1a | 180 | 5 | 5 | 1,4 |
| | 13 | C666C14P+Cl- | Trihexyl (tetradecyl) phosphonium chloride | 5,0 | | | | 1a | 180 | 5 | 5 | 1,4 |
| | 14 | C666C14P+Cl- | Trihexyl (tetradecyl) phosphonium chloride | 6,2 | | | | 1a | 180 | 5 | 5 | 1,4 |
| | 15 | 1-butyl-1-methylPyr+Cl- | 1-methyl-1-butyl pyrrolidinium chloride | 2,3 | | | | 1a | 180 | 5 | 5 | 1,4 |
| | 16 | 1-methyl-triphenylP+Cl- | methyltriphenyl phosphonium chloride | 2,0 | | | | 1a | 240 | 5 | 5 | 1,4 |

EP 3 027 581 B1

| Figure | Experiment (n°) | Ionic Liquid used | | Amount (g) | Additive | | Amount (g) | Reactor type | Temperature (°C) | N2 flow (mL/min) | N2 flow (diluent) (mL/min) | DCE flow (mmol/h) |
|---|---|---|---|---|---|---|---|---|---|---|---|---|
| | | | Name | | Name | | | | | | | |
| Figure 4 | 17 | C4444P+Cl- | Tetrabutyl phosphonium chloride | 1,8 | | | | 1b | 240 | n/a | 5 | 12,7 |
| | 18 | C4444P+Cl- | Tetrabutyl phosphonium chloride | 1,8 | | | | 1b | 240 | n/a | 10 | 25,3 |
| | 19 | C4444P+Cl- | Tetrabutyl phosphonium chloride | 1,8 | | | | 1b | 240 | n/a | 3 | 25,3 |
| | 20 | C4444P+Cl- | Tetrabutyl phosphonium chloride | 3,6 | | | | 1b | 240 | n/a | 3 | 25,3 |
| | 21 | C4444P+Cl- | Tetrabutyl phosphonium chloride | 1,8 | | | | 1b | 240 | n/a | 2 | 38,0 |
| | 22 | C4444P+Cl- | Tetrabutyl phosphonium chloride | 1,8 | CsCl | Caesium chloride | 0,10 | 1b | 240 | n/a | 10 | 25,3 |
| | 23 | C4444P+Cl- | Tetrabutyl phosphonium chloride | 1,8 | BaCl2 | Barium chloride | 0,10 | 1b | 240 | n/a | 10 | 25,3 |
| Figure 5 | 24 | C3333P+Cl- | Tetrapropyl phosphonium chloride | 1,5 | | | | 1a | 240 | 5 | 5 | 1,4 |
| | 25 | C4441P+Cl- | Tributyl methyl phosphonium chloride | 1,5 | | | | 1a | 240 | 5 | 5 | 1,4 |
| | 26 | C4443P+Cl- | tributyl propyl phosphonium chloride | 1,7 | | | | 1a | 240 | 5 | 5 | 1,4 |
| | 27 | C444i4P+Cl- | Tributyl isobutyl phosphonium chloride | 1,8 | | | | 1a | 240 | 5 | 5 | 1,4 |
| | 28 | C4445P+Cl- | Tributyl pentyl phosphonium chloride | 1,9 | | | | 1a | 240 | 5 | 5 | 1,4 |
| | 29 | C3338P+Cl- | Tripropyl octyl phosphonium chloride | 1,9 | | | | 1a | 240 | 5 | 5 | 1,4 |
| | 30 | C4446P+Cl- | Tributyl hexyl phosphonium chloride | 1,9 | | | | 1a | 240 | 5 | 5 | 1,4 |
| | 31 | C4448P+Cl- | Tributyl octyl phosphonium chloride | 2,1 | | | | 1a | 240 | 5 | 5 | 1,4 |
| | 32 | C44410P+Cl- | Tributyl decyl phosphonium chloride | 2,3 | | | | 1a | 240 | 5 | 5 | 1,4 |
| | 33 | C44414P+Cl- | Tributyl tetradecyl phosphonium chloride | 2,5 | | | | 1a | 240 | 5 | 5 | 1,4 |
| Figure 6 | 34 | C4444P+Cl- | Tetrabutyl phosphonium chloride | 0,6 | | | | 2a | 200 | 5 | 5 | 1,4 |
| | 35 | C4444P+Cl- | Tetrabutyl phosphonium chloride | 0,6 | | | | 2a | 240 | 5 | 5 | 1,4 |
| | 36 | C4444P+Cl- | Tetrabutyl phosphonium chloride | 0,15 | | | | 2a | 240 | 5 | 5 | 1,4 |
| Figure 7 | 37 | C4444P+Cl- | Tetrabutyl phosphonium chloride | 1,69 | | | | 2b | 240 | n/a | 3 | 13,6 |

**Claims**

1.  A process for the dehydrohalogenation of a halogenated organic non polymeric compound using a catalyst comprising an ionic liquid having the formula :

    $$[Cat^+] \, [X^-]$$

    wherein :

    $[Cat^+]$ represents one or more organic cationic species, and
    $[X^-]$ represents one or more anionic species.

2.  The process according to claim 1, wherein the cation $[Cat^+]$ is a phosphonium ion and has the following general formula : $[PRaRbRcRd]^+$,
    wherein Ra, Rb, Rc, Rd can be independently chosen to be alkyl (with C1 till C30), aryl (C6 to C10), arylalkyl, heteroaryl, heteroarylalkyl or hydrogen; whereby the alkyl, aryl, arylalkyl, heteroaryl, heteroarylalkyl groups can be substituted with (cyclo)alkyl, (cyclo)alkoxy, aryl, arylalkyl, amide, ammonium or phosphonium groups.

3.  The process according to claim 2, wherein the cation $[Cat^+]$ is chosen from Bu4P$^+$ or [P4,4,4,4]; HBu3P$^+$ or [PH4,4,4]; (C14H29)Bu3P$^+$ or [P14,4,4,4]; and (C14H29)Hex3P$^+$ or [P14,6,6,6].

4.  The process according to claim 3, wherein the cation $[Cat^+]$ is Bu4P$^+$ or [P4,4,4,4] .

5.  The process according to any of claims 1 to 4, wherein the anion $[X^-]$ is the same halide as the one to be eliminated by dehydrohalogenation from the organic compound.

6.  The process according to any of claims 1 to 5, wherein the ionic liquid is supported on a solid carrier like silica, alumina, zirconia or another stable oxide.

7.  The process according to any of claims 1 to 6, wherein the organic non polymeric compound is chosen from chlorinated, fluorinated and brominated non polymeric compounds.

8.  The process according to claim 7, wherein the organic non polymeric compound is a chlorinated one.

9.  The process according to claim 8, wherein the organic non polymeric compound is chosen from TCE (trichloroethane) and DCE (dichloroethane).

10. The process according to claim 9, wherein the organic non polymeric compound is DCE.

**Patentansprüche**

1.  Verfahren zur Dehydrohalogenierung einer halogenierten organischen nichtpolymeren Verbindung unter Verwendung eines Katalysators, der eine ionische Flüssigkeit mit der Formel:

    $$[Cat^+] \, [X^-]$$

    umfasst, wobei:

    $[Cat^+]$ für eine oder mehrere organische kationische Spezies steht und
    $[X^-]$ für eine oder mehrere anionische Spezies steht.

2.  Verfahren nach Anspruch 1, bei dem es sich bei dem Kation $[Cat^+]$ um ein Phosphoniumion mit der allgemeinen Formel $[PRaRbRcRd]^+$ handelt, wobei Ra, Rb, Rc und Rd unabhängig aus Alkyl (mit C1 bis C30), Aryl (C6 bis C10), Arylalkyl, Heteroaryl, Heteroarylalkyl oder Wasserstoff ausgewählt sein können; wobei die Alkyl-, Aryl-, Arylalkyl-, Heteroaryl- und Heteroaryl alkylgruppen durch (Cyclo)alkyl-, (Cyclo)alkoxy-, Aryl-, Arylalkyl-, Amid-, Ammonium- oder Phosphonium gruppen substituiert sein können.

**3.** Verfahren nach Anspruch 2, bei dem das Kation [Cat$^+$] aus Bu4P$^+$ oder [P4,4,4,4]; HBu3P$^+$ oder [PH4,4,4]; (C14H29)Bu3P$^+$ oder [P14,4,4,4] und (C14H29)Hex3P$^+$ oder [P14,6,6,6] ausgewählt ist.

**4.** Verfahren nach Anspruch 3, bei dem es sich bei dem Kation [Cat$^+$] um Bu4P$^+$ oder [P4,4,4,4] handelt.

**5.** Verfahren nach einem der Ansprüche 1 bis 4, bei dem es sich bei dem Anion [X$^-$] um das gleiche Halogenid wie das durch Dehydrohalogenierung aus der organischen Verbindung zu eliminierende Halogenid handelt.

**6.** Verfahren nach einem der Ansprüche 1 bis 5, bei dem die ionische Flüssigkeit auf einem festen Träger wie Siliciumdioxid, Aluminiumoxid, Zirconiumdioxid oder einem anderen stabilen Oxid geträgert ist.

**7.** Verfahren nach einem der Ansprüche 1 bis 6, bei dem die organische nichtpolymere Verbindung aus chlorierten, fluorierten und bromierten nichtpolymeren Verbindungen ausgewählt wird.

**8.** Verfahren nach Anspruch 7, wobei es sich bei der organischen nichtpolymeren Verbindung um eine chlorierte Verbindung handelt.

**9.** Verfahren nach Anspruch 8, bei dem die organische nichtpolymere Verbindung aus TCE (Trichlorethan) und DCE (Dichlorethan) ausgewählt wird.

**10.** Verfahren nach Anspruch 9, bei dem es sich bei der organischen nichtpolymeren Verbindung um DCE handelt.

**Revendications**

**1.** Procédé de déshydrohalogénation d'un composé organique non polymère halogéné au moyen d'un catalyseur comprenant un liquide ionique ayant la formule :

[Cat$^+$][X$^-$]

dans laquelle :

[Cat$^+$] représente une ou plusieurs espèces cationiques organiques, et
[X$^-$] représente une ou plusieurs espèces anioniques.

**2.** Procédé selon la revendication 1, dans lequel le cation [Cat$^+$] est un ion phosphonium et a la formule générale suivante : [PRaRbRcRd]$^+$, dans laquelle Ra, Rb, Rc et Rd peuvent être indépendamment choisis pour être un alkyle (avec C1 jusqu'à C30), un aryle (C6 à C10), un arylalkyle, un hétéroaryle, un hétéroarylalkyle ou un hydrogène ; les groupes alkyle, aryle, arylalkyle, hétéroaryle, hétéroarylalkyle pouvant être substitués par des groupes (cyclo)alkyle, (cyclo)alcoxy, aryle, arylalkyle, amide, ammonium ou phosphonium.

**3.** Procédé selon la revendication 2, dans lequel le cation [Cat$^+$] est choisi parmi Bu4P$^+$ ou [P4,4,4,4] ; HBu3P$^+$ ou [PH4,4,4] ; (C14H29)Bu3P$^+$ ou [P14,4,4,4] ; et (C14H29)Hex3P$^+$ ou [P14,6,6,6].

**4.** Procédé selon la revendication 3, dans lequel le cation [Cat$^+$] est Bu4P$^+$ ou [P4,4,4,4].

**5.** Procédé selon l'une quelconque des revendications 1 à 4, dans lequel l'anion [X$^-$] est le même halogénure que celui devant être éliminé par déshydrohalogénation du composé organique.

**6.** Procédé selon l'une quelconque des revendications 1 à 5, dans lequel le liquide ionique est supporté par un support solide comme la silice, l'alumine, la zircone ou un autre oxyde stable.

**7.** Procédé selon l'une quelconque des revendications 1 à 6, dans lequel le composé organique non polymère est choisi parmi les composés non polymères chlorés, fluorés et bromés.

**8.** Procédé selon la revendication 7, dans lequel le composé organique non polymère est un composé chloré.

**9.** Procédé selon la revendication 8, dans lequel le composé organique non polymère est choisi entre le TCE (trichloroéthane) et le DCE (dichloroéthane).

10. Procédé selon la revendication 9, dans lequel le composé organique non polymère est le DCE.

Figure 1

Figure 2

Figure 3

EP 3 027 581 B1

Figure 4

Figure 5

EP 3 027 581 B1

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHAO.** Dehydrochlorination of polyvinyl chloride in basic ionic liquids. *the 5th ISFR,* 11 October 2009, 260-262
- A highly efficient approach for dehydrochlorinating polyvinyl chloride catalysis by 1-butyl-3-methylimidazolium chloride. *Green Chem.,* 2010, vol. 12, 1062-1065
- **GLAS.** End-of-life treatment of PVC and chlorinated polyethylene by dehydrochlorination in Ionic Liquids. *Chem Sus Chem,* 2014, vol. 7 (2), 610-7
- **S.S. SHAVANOV et al.** *Journal of Organic Chemistry of the USSR,* 1990, vol. 26 (5), 643-647